(19) Europäisches Patentamt
European Patent Office
Office européen des brevets

(11) Veröffentlichungsnummer: **0 584 655 A2**

(12) # EUROPÄISCHE PATENTANMELDUNG


(21) Anmeldenummer: **93112877.1**

(22) Anmeldetag: **11.08.93**

(51) Int. Cl.[5]: **C07D 251/34**, A01N 43/64, C07D 251/38, //C07C275/70, C07C335/38

(30) Priorität: **24.08.92 DE 4228000**

(43) Veröffentlichungstag der Anmeldung: **02.03.94 Patentblatt 94/09**

(84) Benannte Vertragsstaaten: **BE CH DE ES FR GB IT LI NL**



(71) Anmelder: **BAYER AG**

**D-51368 Leverkusen(DE)**

(72) Erfinder: **Schallner, Otto**
**Noldeweg 22**
**D-40789 Monheim(DE)**
Erfinder: **Lürssen, Klaus**
**August-Kierspel-Strasse 145**
**D-51469 Bergisch Gladbach(DE)**
Erfinder: **Santel, Hans-Joachim**
**Grünstrasse 9a**
**D-51371 Leverkusen(DE)**
Erfinder: **Schmidt, Rudolf R.**
**Im Waldwinkel 110**
**D-51467 Bergisch Gladbach(DE)**
Erfinder: **Vosswinkel, Renate**
**Sankt-Maternus-Eck 14a**
**D-51515 Kürten-Bechen(DE)**


(54) **3-Aryl-triazin-2,4-dione als Herbizide.**


(57) Die Erfindung betrifft neue 3-Aryl-triazin-2,4-dione der allgemeinen Formel (I),

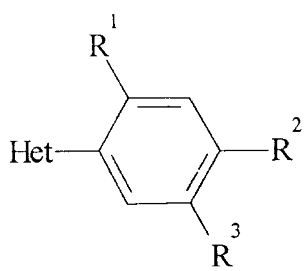

(I)

in welcher
Het für einen Rest der Formel

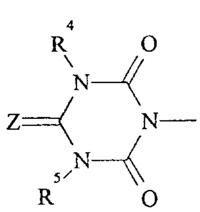

oder

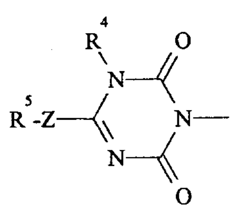


EP 0 584 655 A2

steht,

$R^1$ für Wasserstoff oder Halogen steht,
$R^2$ für Halogen, Cyano oder Nitro steht und
$R^3$ für einen Rest der Formel $-X-R^6$ oder $-CO-X-R^7$ steht, wobei
$R^4$ für Wasserstoff, Cyano, Alkyl, Alkenyl, Alkinyl oder Halogenalkyl steht,
$R^5$ für Alkyl, Alkenyl oder Alkinyl steht,
$R^6$ für Wasserstoff oder für gegebenenfalls substituiertes Alkyl steht,
$R^7$ für Wasserstoff oder für gegebenenfalls substituiertes Alkyl steht,
X für Sauerstoff oder Schwefel steht und
Z für Sauerstoff oder Schwefel steht,

ein Verfahren zu ihrer Herstellung, neue Zwischenprodukte sowie ihre Verwendung als Herbizide.

Die Erfindung betrifft neue 3-Aryl-triazin-2,4-dione, ein Verfahren zu ihrer Herstellung, neue Zwischenprodukte sowie ihre Verwendung als Herbizide.

Es ist bekannt, daß bestimmte Triazin-2,4-dione wie beispielsweise die Verbindung 3-Isopropyl-6-[N-acetyl-N-(2,2-dimethyl-1-propyl)-amino]-(3H,5H)-1,3,5-triazin-2,4-dion herbizide Eigenschaften besitzen (vergl. z.B. DE 26 03 180).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue 3-Aryl-triazin-2,4-dione der allgemeinen Formel (I),

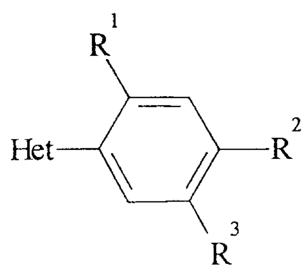

(I)

in welcher

Het für einen Rest der Formel

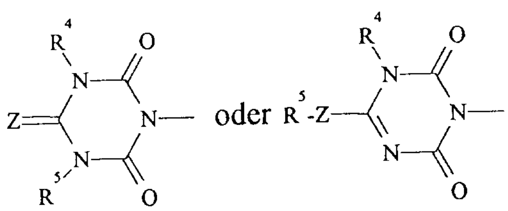

steht,

- $R^1$ für Wasserstoff oder Halogen steht,
- $R^2$ für Halogen, Cyano oder Nitro steht und
- $R^3$ für einen Rest der Formel $-X-R^6$ oder $-CO-X-R^7$ steht, wobei
- $R^4$ für Wasserstoff, Cyano, Alkyl, Alkenyl, Alkinyl oder Halogenalkyl steht,
- $R^5$ für Alkyl, Alkenyl oder Alkinyl steht,
- $R^6$ für Wasserstoff oder für gegebenenfalls substituiertes Alkyl steht,
- $R^7$ für Wasserstoff oder für gegebenenfalls substituiertes Alkyl steht,
- X für Sauerstoff oder Schwefel steht und
- Z für Sauerstoff oder Schwefel steht,

gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen 3-Aryl-triazin-2,4-dione der allgemeinen Formel (I),

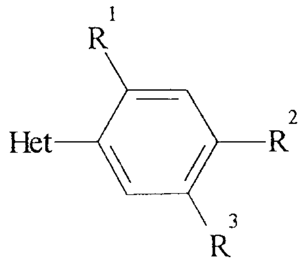

(I)

in welcher
Het für einen Rest der Formel

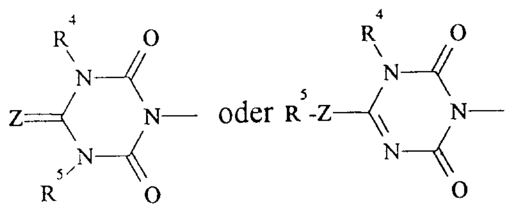

steht,

- $R^1$ für Wasserstoff oder Halogen steht,
- $R^2$ für Halogen, Cyano oder Nitro steht und
- $R^3$ für einen Rest der Formel $-X-R^6$ oder $-CO-X-R^7$ steht, wobei
- $R^4$ für Wasserstoff, Cyano, Alkyl, Alkenyl, Alkinyl oder Halogenalkyl steht,
- $R^5$ für Alkyl, Alkenyl oder Alkinyl steht,
- $R^6$ für Wasserstoff oder für gegebenenfalls substituiertes Alkyl steht,
- $R^7$ für Wasserstoff oder für gegebenenfalls substituiertes Alkyl steht,
- X für Sauerstoff oder Schwefel steht und
- Z für Sauerstoff oder Schwefel steht,

erhält, wenn man substituierte Phenylharnstoff-Derivate der allgemeinen Formel (II),

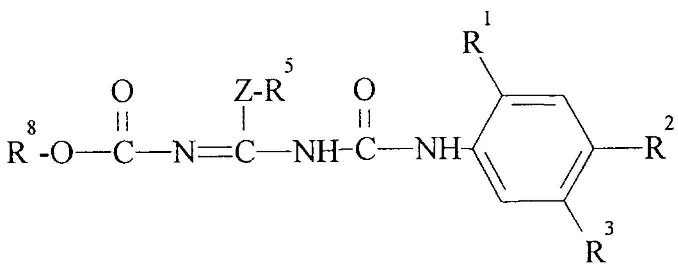

(II)

in welcher
$R^1$, $R^2$, $R^3$, $R^5$ und Z die oben angegebenen Bedeutungen haben und
$R^8$ für Alkyl oder Aryl steht,

gegebenenfalls in Gegenwart eines Verdünnungsmittels und gegebenenfalls in Gegenwart eines Reaktionshilfsmittels cyclisiert und gegebenenfalls in einer anschließendenden 2.Stufe die so erhältlichen 3-Aryl-(1H)-triazin-2,4-dione der Formel (Ia),

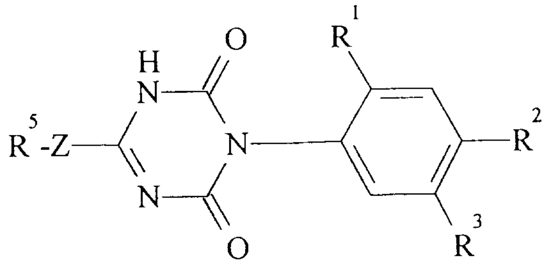

(Ia)

in welcher

$R^1$, $R^2$, $R^3$, $R^5$ und Z die die oben angegebenen Bedeutungen haben mit Alkylierungsmitteln der Formel (III),

$R^{4-1}$-E (III)

in welcher

$R^{4-1}$ für Alkyl, Alkenyl, Alkinyl oder Halogenalkyl steht und

E für eine elektronenanziehende Abgangsgruppe steht,

oder mit Bromcyan gegebenenfalls in Gegenwart eines Verdünnungsmittels und gegebenenfalls in Gegenwart eines Reaktionshilfsmittels umsetzt und gegebenenfalls in einer anschließendenden 3.Stufe die so erhältlichen 3-Aryl-triazin-2,4-dione der Formel (Ib),

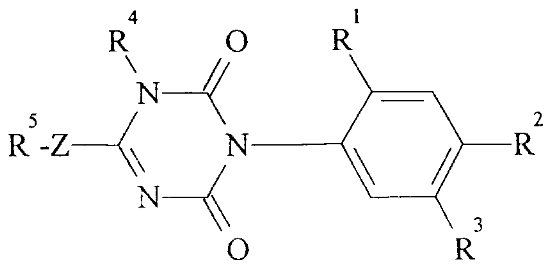

(Ib)

in welcher

$R^1$, $R^2$, $R^3$, $R^4$, $R^5$ und Z die die oben angegebenen Bedeutungen haben,

gegebenenfalls in Gegenwart eines Verdünnungsmittels und gegebenenfalls in Gegenwart eines Reaktionshilfsmittels thermisch isomerisiert.

Schließlich wurde gefunden, daß die neuen 3-Aryl-triazin-2,4-dione der allgemeinen Formel (I) herbizide Eigenschaften besitzen.

Überraschenderweise zeigen die erfindungsgemäßen 3-Aryl-triazin-2,4-dione der allgemeinen Formel (I) eine erheblich bessere herbizide Wirksamkeit gegenüber Problemunkräutern und gleichzeitig eine erheblich bessere Verträglichkeit gegenüber wichtigen Kulturpflanzen im Vergleich zu den aus dem Stand der Technik bekannten Triazin-2,4-dionen, wie beispielsweise die Verbindung 3-Isopropyl-6-[N-acetyl-N-(2,2-dimethyl-1-propyl)-amino]-(3H,5H)-1,3,5-triazin-2,4-dion, welche chemisch und wirkungsmäßig naheliegende Verbindungen sind.

Die erfindungsgemäßen 3-Aryl-triazin-2,4-dione sind durch die Formel (I) allgemein definiert. Bevorzugt sind Verbindungen der Formel (I), bei welchen Het für einen Rest der Formel

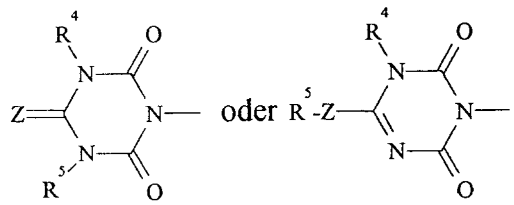

steht,

$R^1$ für Wasserstoff, Fluor, Chlor Brom oder Iod steht,

$R^2$ für Fluor, Chlor Brom, Iod, Cyano oder Nitro steht,

$R^3$ für einen Rest der Formel $-X-R^6$ oder $-CO-X-R^7$ steht, wobei

$R^4$ für Wasserstoff, Cyano, für geradkettiges oder verzweigtes Alkyl mit 1 bis 8 Kohlenstoffatomen, für geradkettiges oder verzweigtes Alkenyl mit 2 bis 8 Kohlenstoffatomen, für geradkettiges oder verzweigtes Alkinyl mit 2 bis 8 Kohlenstoffatomen oder für geradkettiges oder verzweigtes Halogenalkyl mit 1 bis 6 Kohlenstoffatomen und 1 bis 13 gleichen oder verschiedenen Halogenatomen - insbesondere Fluor, Chlor, Brom und/oder Iod - steht,

$R^5$ für geradkettiges oder verzweigtes Alkyl mit 1 bis 8 Kohlenstoffatomen, für geradkettiges oder verzweigtes Alkenyl mit 2 bis 8 Kohlenstoffatomen oder für geradkettiges oder verzweigtes Alkinyl mit 2 bis 8 Kohlenstoffatomen steht,

$R^6$ jeweils für Wasserstoff, für geradkettiges oder verzweigtes Alkyl mit 1 bis 8 Kohlenstoffatomen, für geradkettiges oder verzweigtes Halogenalkyl mit 1 bis 6 Kohlenstoffatomen und 1 bis 13 gleichen oder verschiedenen Halogenatomen - insbesondere Fluor, Chlor, Brom und/oder Iod -, für jeweils geradkettiges oder verzweigtes Alkoxyalkyl, Alkoxycarbonylalkyl, Alkylcarbonyloxyalkyl, Alkylthiocarbonylalkyl oder Alkylcarbonylthioalkyl mit jeweils 1 bis 6 Kohlenstoffatomen in den einzelnen Alkylteilen oder für gegebenenfalls einfach oder mehrfach, gleich oder verschieden substituiertes Arylalkyl mit 6 oder 10 Kohlenstoffatomen im Arylteil und 1 bis 6 Kohlenstoffatomen im geradkettigen oder verzweigten Alkylteil stehen, wobei als Arylsubstituenten infrage kommen: Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl und/oder geradkettiges oder verzweigtes Alkoxy und/oder geradkettiges oder verzweigtes Halogenalkyl und/oder geradkettiges oder verzweigtes Halogenalkoxy mit jeweils 1 bis 4 Kohlenstoffatomen und gegebenenfalls 1 bis 9 gleichen oder verschiedenen Halogenatomen substituiertes Phenyl,

$R^7$ jeweils für Wasserstoff, für geradkettiges oder verzweigtes Alkyl mit 1 bis 8 Kohlenstoffatomen, für geradkettiges oder verzweigtes Halogenalkyl mit 1 bis 6 Kohlenstoffatomen und 1 bis 13 gleichen oder verschiedenen Halogenatomen - insbesondere Fluor, Chlor, Brom und/oder Iod -, für jeweils geradkettiges oder verzweigtes Alkoxyalkyl, Alkoxycarbonylalkyl, Alkylcarbonyloxyalkyl, Alkylthiocarbonylalkyl oder Alkylcarbonylthioalkyl mit jeweils 1 bis 6 Kohlenstoffatomen in den einzelnen Alkylteilen oder für gegebenenfalls einfach oder mehrfach, gleich oder verschieden substituiertes Arylalkyl mit 6 oder 10 Kohlenstoffatomen im Arylteil und 1 bis 6 Kohlenstoffatomen im geradkettigen oder verzweigten Alkylteil stehen, wobei als Arylsubstituenten infrage kommen: Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl und/oder geradkettiges oder verzweigtes Alkoxy und/oder geradkettiges oder verzweigtes Halogenalkyl und/oder geradkettiges oder verzweigtes Halogenalkoxy mit jeweils 1 bis 4 Kohlenstoffatomen und gegebenenfalls 1 bis 9 gleichen oder verschiedenen Halogenatomen substituiertes Phenyl,

X für Sauerstoff oder Schwefel steht und

Z für Sauerstoff oder Schwefel steht.

Besonders bevorzugt sind Verbindungen der Formel (I), bei welchen Het für einen Rest der Formel

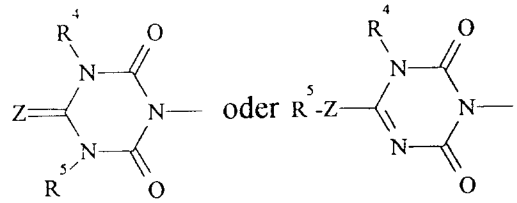

steht,

$R^1$ für Wasserstoff, Fluor, Chlor oder Brom steht,

$R^2$ für Fluor, Chlor, Brom, Cyano oder Nitro steht,

$R^3$ für einen Rest der Formel $-X-R^6$ oder $-CO-X-R^7$ steht, wobei

$R^4$ für Wasserstoff, Cyano, für geradkettiges oder verzweigtes Alkyl mit 1 bis 6 Kohlenstoffatomen, für geradkettiges oder verzweigtes Alkenyl mit 2 bis 6 Kohlenstoffatomen, für geradkettiges oder verzweigtes Alkinyl mit 2 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom - steht,

$R^5$ für geradkettiges oder verzweigtes Alkyl mit 1 bis 6 Kohlenstoffatomen, für geradkettiges oder verzweigtes Alkenyl mit 2 bis 6 Kohlenstoffatomen oder für geradkettiges oder verzweigtes Alkinyl mit 2 bis 6 Kohlenstoffatomen steht,

$R^6$ für Wasserstoff, für geradkettiges oder verzweigtes Alkyl mit 1 bis 6 Kohlenstoffatomen, für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom -, für jeweils geradkettiges oder verzweigtes Alkoxyalkyl, Alkoxycarbonylalkyl, Alkylcarbonyloxyalkyl, Alkylthiocarbonylalkyl oder Alkylcarbonylthioalkyl mit jeweils 1 bis 4 Kohlenstoffatomen in den einzelnen Alkylteilen oder für gegebenenfalls einfach bis fünffach, gleich oder verschieden substituiertes Phenylalkyl mit 1 bis 4 Kohlenstoffatomen im geradkettigen oder verzweigten Alkylteil stehen, wobei als Phenylsubstituenten infrage kommen:

Halogen, Cyano, Nitro, jeweils geradkettiges oder verzweigtes Alkyl, Alkoxy, Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach oder mehrfach, gleich oder verschieden durch Halogen und/oder geradkettiges oder verzweigtes Alkyl und/oder geradkettiges oder verzweigtes Alkoxy und/oder geradkettiges oder verzweigtes Halogenalkyl und/oder geradkettiges oder verzweigtes Halogenalkoxy mit jeweils 1 bis 3 Kohlenstoffatomen und gegebenenfalls 1 bis 7 gleichen oder verschiedenen Halogenatomen substituiertes Phenyl,

$R^7$ für Wasserstoff für geradkettiges oder verzweigtes Alkyl mit 1 bis 6 Kohlenstoffatomen, für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom -, für jeweils geradkettiges oder verzweigtes Alkoxyalkyl, Alkoxycarbonylalkyl, Alkylcarbonyloxyalkyl, Alkylthiocarbonylalkyl oder Alkylcarbonylthioalkyl mit jeweils 1 bis 4 Kohlenstoffatomen in den einzelnen Alkylteilen oder für gegebenenfalls einfach bis fünffach, gleich oder verschieden substituiertes Phenylalkyl mit 1 bis 4 Kohlenstoffatomen im geradkettigen oder verzweigten Alkylteil stehen, wobei als Phenylsubstituenten infrage kommen:

Halogen, Cyano, Nitro, jeweils geradkettiges oder verzweigtes Alkyl, Alkoxy, Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach oder mehrfach, gleich oder verschieden durch Halogen und/oder geradkettiges oder verzweigtes Alkyl und/oder geradkettiges oder verzweigtes Alkoxy und/oder geradkettiges oder verzweigtes Halogenalkyl und/oder geradkettiges oder verzweigtes Halogenalkoxy mit jeweils 1 bis 3 Kohlenstoffatomen

und gegebenenfalls 1 bis 7 gleichen oder verschiedenen Halogenatomen substituiertes Phenyl,

X für Sauerstoff oder Schwefel steht und

Z für Sauerstoff oder Schwefel steht.

Ganz besonders bevorzugt sind Verbindungen der Formel (I), bei welchen Het für einen Rest der Formel

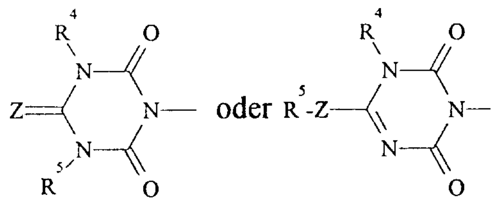

steht,

$R^1$ für Wasserstoff, Fluor oder Chlor steht,

$R^2$ für Fluor, Chlor, Cyano oder Nitro steht,

$R^3$ für einen Rest der Formel $-X-R^6$ oder $-CO-X-R^7$ steht, wobei

$R^4$ für Wasserstoff, Cyano, für geradkettiges oder verzweigtes Alkyl mit 1 bis 4 Kohlenstoffatomen, für geradkettiges oder verzweigtes Alkenyl mit 2 bis 4 Kohlenstoffatomen, für geradkettiges oder verzweigtes Alkinyl mit 2 bis 4 Kohlenstoffatomen oder für geradkettiges oder verzweigtes Halogenalkyl mit 1 bis 3 Kohlenstoffatomen und 1 bis 7 gleichen oder verschiedenen Halogenatomen - insbesondere Fluor, Chlor und/oder Brom - steht,

$R^5$ für geradkettiges oder verzweigtes Alkyl mit 1 bis 4 Kohlenstoffatomen, für geradkettiges oder verzweigtes Alkenyl mit 2 bis 4 Kohlenstoffatomen oder für geradkettiges oder verzweigtes Alkinyl mit 2 bis 4 Kohlenstoffatomen steht,

$R^6$ für Wasserstoff, für geradkettiges oder verzweigtes Alkyl mit 1 bis 4 Kohlenstoffatomen, für geradkettiges oder verzweigtes Halogenalkyl mit 1 bis 3 Kohlenstoffatomen und 1 bis 7 gleichen oder verschiedenen Halogenatomen - insbesondere Fluor, Chlor oder Brom -, für jeweils geradkettiges oder verzweigtes Alkoxyalkyl, Alkoxycarbonylalkyl, Alkylcarbonyloxyalkyl, Alkylthiocarbonylalkyl oder Alkylcarbonylthioalkyl mit jeweils 1 bis 3 Kohlenstoffatomen in den einzelnen Alkylteilen oder für gegebenenfalls einfach bis dreifach, gleich oder verschieden substituiertes Phenylalkyl mit 1 bis 2 Kohlenstoffatomen im Alkylteil stehen, wobei als Phenylsubstituenten infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Ethylsulfinyl, Methylsulfonyl, Ethylsulfonyl, Trifluormethyl, Difluormethyl, Trifluormethoxy, Difluormethoxy, Trifluormethylthio, Trifluormethylsulfinyl, Trifluormethylsulfonyl, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl, Ethyl, n- oder i-Propyl, Methoxy, Ethoxy, Trifluormethyl und/oder Trifluormethoxy substituiertes Phenyl,

$R^7$ für Wasserstoff, für geradkettiges oder verzweigtes Alkyl mit 1 bis 4 Kohlenstoffatomen, für geradkettiges oder verzweigtes Halogenalkyl mit 1 bis 3 Kohlenstoffatomen und 1 bis 7 gleichen oder verschiedenen Halogenatomen - insbesondere Fluor, Chlor oder Brom -, für jeweils geradkettiges oder verzweigtes Alkoxyalkyl, Alkoxycarbonylalkyl, Alkylcarbonyloxyalkyl, Alkylthiocarbonylalkyl oder Alkylcarbonylthioalkyl mit jeweils 1 bis 3 Kohlenstoffatomen in den einzelnen Alkylteilen oder für gegebenenfalls einfach bis dreifach, gleich oder verschieden substituiertes Phenylalkyl mit 1 bis 2 Kohlenstoffatomen im Alkylteil stehen, wobei als Phenylsubstituenten infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Ethylsulfinyl, Methylsulfonyl, Ethylsulfonyl, Trifluormethyl, Difluormethyl, Trifluormethoxy, Difluormethoxy, Trifluormethylthio, Trifluormethylsulfinyl, Trifluormethylsulfonyl, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl, Ethyl, n- oder i-Propyl, Methoxy, Ethoxy, Trifluormethyl und/oder Trifluormethoxy substituiertes Phenyl,

X für Sauerstoff oder Schwefel steht und

Z für Sauerstoff oder Schwefel steht.

Im einzelnen seien auf die bei den Herstellungsbeispielen genannten Verbindungen verwiesen.

Verwendet man beispielsweise N-(4-Chlor-2-fluor-5-methoxycarbonyl-phenyl)-N'-(1-ethoxy-1-methoxycarbonylimino-methyl)-harnstoff als Ausgangsverbindung und Methyliodid als Alkylierungsmittel, so läßt sich der Reaktionsablauf des erfindungsgemäßen Verfahrens durch das folgende Formelschema darstellen:

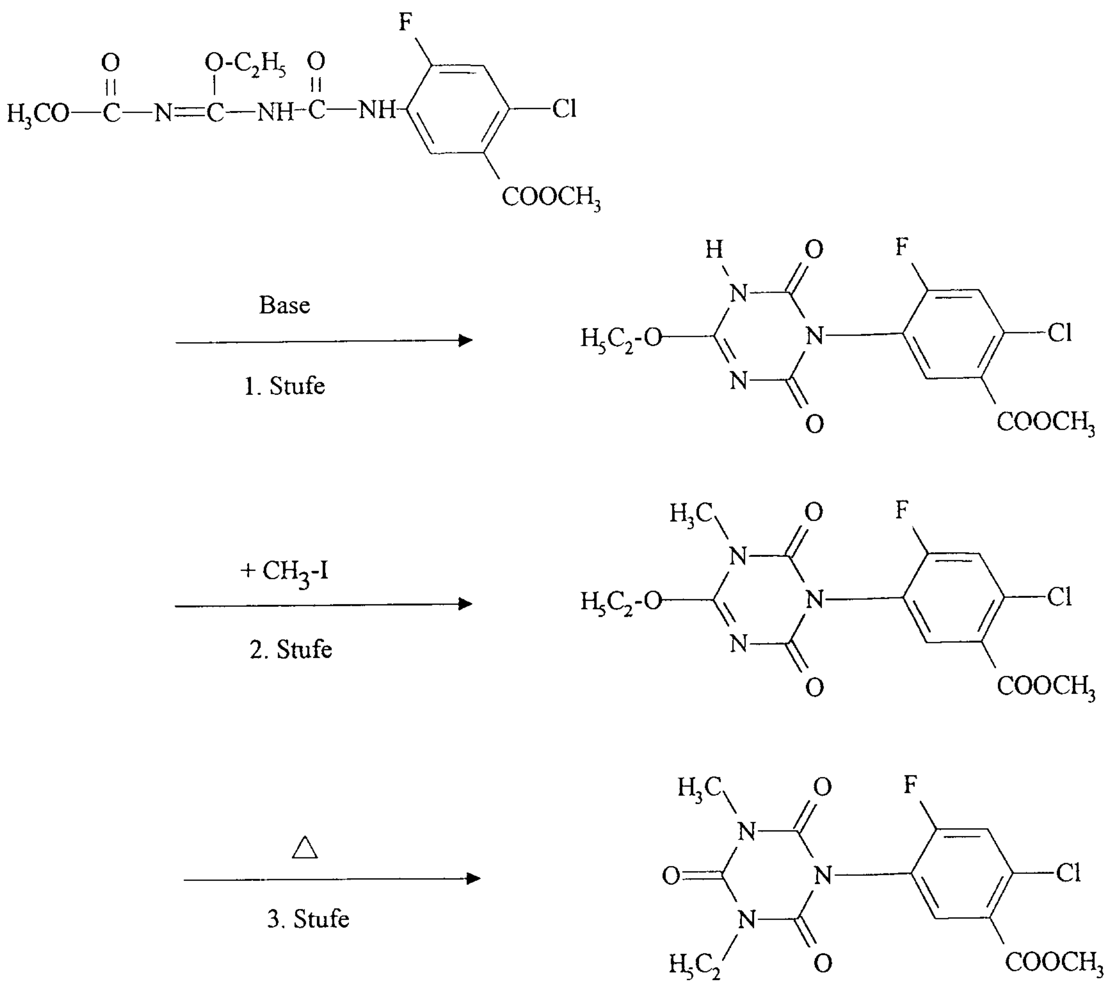


Die zur Durchführung des erfindungsgemäßen Verfahrens als Ausgangsstoffe benötigten substituierten Phenylharnstoff-Derivate sind durch die Formel (II) allgemein definiert. In dieser Formel (II) stehen $R^1$, $R^2$, $R^3$, $R^5$ und Z vorzugsweise für diejenigen Reste, die bereits im Zusammenhang mit der Beschreibung der erfindungsgemäßen Verbindungen der Formel (I) als bevorzugt für diese Substituenten genannt wurden. $R^8$ steht vorzugsweise für geradkettiges oder verzweigtes Alkyl mit 1 bis 6 Kohlenstoffatomen oder für Phenyl, insbesondere für Methyl oder Ethyl oder Phenyl.

Die substituierten Phenylharnstoff-Derivate der Formel (II) sind noch nicht bekannt und ebenfalls Gegenstand der Erfindung. Man erhält sie, wenn man Phenylisocyanate der Formel (IV),

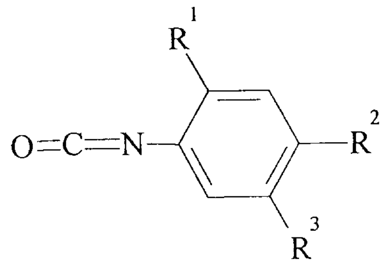

(IV)

in welcher

$R^1$, $R^2$ und $R^3$ die oben angegebenen Bedeutungen haben,

mit Carbaminsäure-Derivaten der Formel (V),

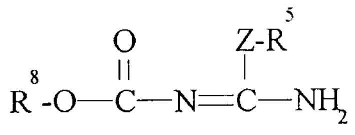

(V)

in welcher

$R^5$, $R^8$ und Z die oben angegebenen Bedeutungen haben,

gegebenenfalls in Gegenwart eines Verdünnungsmittels wie beispielsweise Dichlormethan bei Temperaturen zwischen 0 °C und 50 °C umsetzt. Dabei ist es auch möglich, die als Ausgangsverbindungen benötigten Phenylisocyanate der Formel (IV) in einer vorgelagerten Reaktion in allgemein üblicher Art und Weise aus entsprechenden Anilinen und Phosgen oder Diphosgen herzustellen und ohne Isolierung direkt im Reaktionsgefäß mit den Carbaminsäure-Derivaten der Formel (V) weiter umzusetzen (vergleiche hierzu auch die Herstellungsbeispiele).

Die Phenylisocyanate der Formel (IV) sind allgemein bekannte Verbindungen der organischen Chemie oder erhältlich in Analogie zu allgemein bekannten Verfahren (vergl. z.B. GB 14 35 585).

Die Carbaminsäure-Derivate der Formel (V) sind ebenfalls allgemein bekannte Verbindungen der organischen Chemie (vergl. z.B. DE 29 33 889; Liebigs Ann. Chem. 1985, 2363-2370).

Die zur Durchführung des erfindungsgemäßen Verfahrens weiterhin als Ausgangsstoffe benötigten Alkylierungsmittel sind durch die Formel (III) allgemein definiert. In dieser Formel (III) steht $R^{4-1}$ vorzugsweise für geradkettiges oder verzweigtes Alkyl mit 1 bis 6 Kohlenstoffatomen, für geradkettiges oder verzweigtes Alkenyl mit 2 bis 6 Kohlenstoffatomen, für geradkettiges oder verzweigtes Alkinyl mit 2 bis 6 Kohlenstoffatomen oder für geradkettiges oder verzweigtes Halogenalkyl mit 1 bis 6 Kohlenstoffatomen und 1 bis 13 gleichen oder verschiedenen Halogenatomen, insbesondere Fluor, Chlor, Brom und /oder Iod. $R^{4-1}$ steht insbesondere für geradkettiges oder verzweigtes Alkyl mit 1 bis 4 Kohlenstoffatomen, für geradkettiges oder verzweigtes Alkenyl mit 2 bis 4 Kohlenstoffatomen, für geradkettiges oder verzweigtes Alkinyl mit 2 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen, insbesondere Fluor, Chlor und /oder Brom.

E steht für einen bei Alkylierungsmitteln üblichen Abgangsrest, vorzugsweise für Halogen, insbesondere für Chlor, Brom oder Iod oder für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy.

Die Alkylierungsmittel der Formel (III) sind ebenfalls allgemein bekannte Verbindungen der organischen Chemie.

Als Verdünnungsmittel zur Durchführung der 1. Stufe des erfindungsgemäßen Verfahrens kommen alle üblichen organischen oder anorganischen Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder - diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid oder Alkohole, wie Methanol, Ethanol, Propanol oder Butanol.

Die 1. Stufe des erfindungsgemäßen Verfahrens kann gegebenenfalls in Gegenwart eines geeigneten basischen Reaktionshilfsmittels durchgeführt werden. Als solche kommen alle üblichen anorganischen oder organischen Basen infrage. Hierzu gehören beispielsweise Erdalkali- oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der 1. Stufe des erfindungsgemäßen Verfahrens in einem größeren Bereich variiert werden. Im allgemeinen arbeitet man bei Temperaturen zwischen 0 °C und 120 °C, vorzugsweise bei Temperaturen zwischen 20 °C und 80 °C.

Die 1. Stufe des erfindungsgemäßen Verfahren wird üblicherweise unter Normaldruck durchgeführt. Es ist jedoch auch möglich unter erhöhtem oder vermindertem Druck zu arbeiten.

Zur Durchführung der 1. Stufe des erfindungsgemäßen Verfahrens setzt man pro Mol substituiertem Phenylharnstoff-Derivat der Formel (II) im allgemeinen 0,01 bis 2,0 Mol, vorzugsweise 0,1 bis 1,0 Mol Base als Reaktionshilfsmittel ein. Die Reaktionsdurchführung, Aufarbeitung und Isolierung der Reaktionsprodukte erfolgt in Analogie zu bekannten Verfahren (vergl. hierzu auch die Herstellungsbeispiele).

Als Verdünnungsmittel zur Durchführung der 2. Stufe des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide, wie Dimethylsulfoxid.

Die 2. Stufe des erfindungsgemäßen Verfahrens wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die 2. Stufe des erfindungsgemäßen Verfahrens kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributylmethylphosphoniumbromid,Trimethyl-$C_{13}$/$C_{15}$-alkylammoniumchlorid, Trimethyl-$C_{13}$/$C_{15}$-alkylammoniumbromid, Dibenzyl-dimethyl-ammoniummethylsulfat, Dimethyl-$C_{12}$/$C_{14}$-alkyl-benzylammoniumchlorid, Dimethyl-$C_{12}$/$C_{14}$-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Die Reaktionstemperaturen können bei der Durchführung der 2. Stufe des erfindungsgemäßen Verfahrens in einem größeren Bereich variiert werden. Im allgemeinen arbeitet man bei Temperaturen zwischen -20 °C und +120 °C, vorzugsweise bei Temperaturen zwischen 0 °C und 80 °C.

Die 2. Stufe des erfindungsgemäßen Verfahrens wird üblicherweise unter Normaldruck durchgeführt. Es ist jedoch auch möglich unter erhöhtem oder vermindertem Druck zu arbeiten.

Zur Durchführung der 2. Stufe des erfindungsgemäßen Verfahrens setzt man pro Mol 3-Aryl-(1H)-triazin-2,4-dion der Formel (Ia) im allgemeinen 1,0 bis 5,0 Mol, vorzugsweise 1,0 bis 2,5 Mol an Alkylierungsmittel der Formel (III) oder alternativ an Bromcyan, 1,0 bis 5,0 Mol, vorzugsweise 1,0 bis 2,5 Mol Base als Reaktionshilfsmittel und gegebenenfalls 0,001 bis 2,0 Mol, vorzugsweise 0,001 bis 1,0 Mol Phasentransfer-Katalysator ein.

Die Reaktionsdurchführung, Aufarbeitung und Isolierung der Reaktionsprodukte erfolgt in Analogie zu bekannten Verfahren (vergl. hierzu auch die Herstellungsbeispiele).

Als Verdünnungsmittel zur Durchführung der 3. Stufe des erfindungsgemäßen Verfahrens kommen übliche inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide, wie Dimethylsulfoxid.

Die 3. Stufe des erfindungsgemäßen Verfahrens kann gegebenenfalls auch in Gegenwart eines geeigneten basischen Reaktionshilfsmittels durchgeführt werden. Als solche kommen alle üblichen anorganischen oder organischen Basen infrage. Hierzu gehören beispielsweise Erdalkali- oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der 3. Stufe des erfindungsgemäßen Verfahrens in einem größeren Bereich variiert werden. Im allgemeinen arbeitet man bei Temperaturen zwischen 50 ° C und 200 ° C, vorzugsweise bei Temperaturen zwischen 80 ° C und 150 ° C.

Die 3. Stufe des erfindungsgemäßen Verfahren wird üblicherweise unter Normaldruck durchgeführt. Es ist jedoch auch möglich unter erhöhtem oder vermindertem Druck zu arbeiten.

Zur Durchführung der 3. Stufe des erfindungsgemäßen Verfahrens setzt man pro Mol substituiertem 3-Aryl-triazin-2,4-dion der Formel (Ib) im allgemeinen 0,01 bis 2,0 Mol, vorzugsweise 0,1 bis 1,0 Mol Base als Reaktionshilfsmittel ein, es ist jedoch auch möglich, die 3. Stufe des erfindungsgemäßen Verfahrens ohne den Zusatz eines basischen Reaktionshilfsmittels durchzuführen. Die Reaktionsdurchführung, Aufarbeitung und Isolierung der Reaktionsprodukte erfolgt in Analogie zu bekannten Verfahren (vergl. hierzu auch die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe des Brechungsindex oder der Protonen-Kernresonanzspektroskopie ($^1$H-NMR).

Die erfindungsgemäßen Wirkstoffe können als Defoliants, Desiccants, Krautabtötungsmittel und insbesondere als Unkrautvernichtungsmittel verwendet werden.Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dacryloctenium, Agrostis, Alopecurus, Apera.

Monokotyle Kulturen der Gattungen: Oryza Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen, auf Zier- und

Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von mono- und dikotylen Unkräutern in monokotylen und dikotylen Kulturen wie beispielsweise Mais, Weizen, Zuckerrüben und Soja einsetzen.

Daneben greifen die erfindungsgemäßen Wirkstoffe auch in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen von dem Zeitpunkt der Anwendung bezogen auf das Entwicklungstadium der Pflanze sowie von den auf die Pflanzen oder ihre Umgebung ausgebrachten Wirkstoffmengen und von der Art der Applikation ab. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle, ist aber auch in anderen Kulturen, wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Daneben besitzen die erfindungsgemäßen Wirkstoffe in entsprechenden Aufwandmengen auch fungizide Wirksamkeit und können unter anderem zur Bekämpfung von Reiskrankheiten wie beispielsweise gegen den Erreger der Reisfleckenkrankheit (Pyricularia oryzae) eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser.

Als feste Trägerstoffe kommen infrage: z.B. Ammoniumsalze und natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba oder Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen; Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro Hektar.

Die Herstellung und die Verwendung der erfindungsgemäßen Wirkstoffe geht aus den nachfolgenden Beispielen hervor.

Die Herstellung und die Verwendung der erfindungsgemäßen Wirkstoffe geht aus den nachfolgenden Beispielen hervor.

Herstellungsbeispiele:

Beipiel 1:

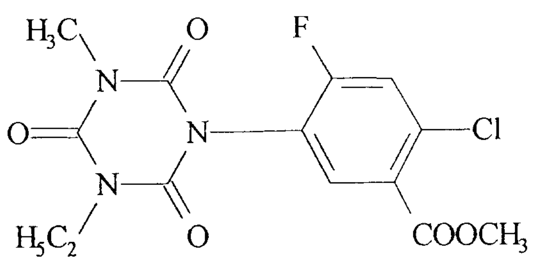

Eine Lösung von 1,0 g (0,0028 Mol) 3-(4-Chlor-2-fluor-5-methoxycarbonyl-phenyl)-1-methyl-6-ethoxy-(1H,3H)-13,5-triazin-2,4-dion in 40 ml Toluol wird 16 Stunden auf Rückflußtemperatur erhitzt. Zur Aufarbeitung wird das Lösungsmittel im Vakuum abdestilliert und der Rückstand im Hochvakuum getrocknet.

Man erhält 0,8 g (80 % der Theorie) 3-(4-Chlor-2-fluor-5-methoxycarbonylphenyl)-1-methyl-5-ethyl-1,3,5-triazin-2,4,6-trion mit Schmelzpunkt 48-49 °C.

Beispiel 2:

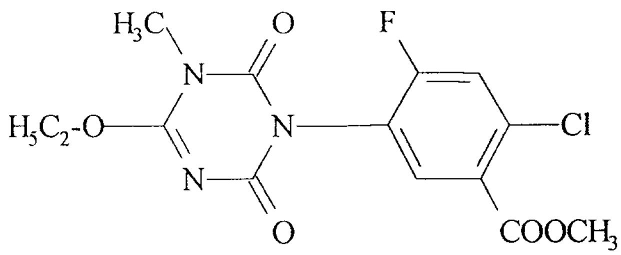

Zu einer Lösung von 2,9 g (0,0085 Mol) 3-(4-Chlor-2-fluor-5-methoxycarbonylphenyl)-6-ethoxy-(1H,3H)-1,3,5-triazin-2,4-dion in 100 ml trockenem Acetonitril gibt man bei Raumtemperatur nacheinander 1,3 g (0,009 Mol) Kaliumcarbonat und 1,3 g (0,009 Mol) Methyliodid und rührt anschließend 18 Stunden bei Raumtemperatur. Zur Aufarbeitung gibt man die Reaktionsmischung in Wasser, säuert mit Salzsäure an und extrahiert mit Essigester. Die organische Phase wird getrocknet und das Lösungsmittel im Vakuum abdestilliert.

Man erhält 1,3 g (43 % der Theorie) 3-(4-Chlor-2-fluor-5-methoxycarbonyl-phenyl)-1-methyl-6-ethoxy-(1H,3H)-1,3,5-triazin-2,4-dion mit Schmelzpunkt 45-47 ° C.

Beispiel 3:

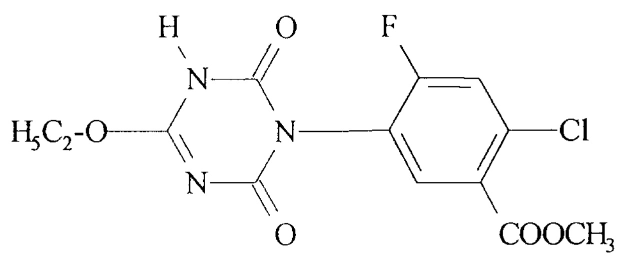

Zu einer Lösung von 4,5 g (0,012 Mol) N-(4-Chlor-2-fluor-5-methoxycarbonylphenyl)-N'-(1-ethoxy-1-methoxycarbonylimino-methyl)-harnstoff in 150 ml Methanol gibt man bei Raumtemperatur 0,7 g (0,002 Mol) Natriummethylat und erwärmt anschließend für 16 Stunden bei Rückflußtemperatur. Zur Aufarbeitung wird das Lösungsmittel im Vakuum abdestilliert, der Rückstand in Eiswasser gegeben, mit Salzsäure angesäuert. Der ausgefallene Feststoff wird abgesaugt, mit Wasser gewaschen und im Vakuum getrocknet.

Man erhält 2,9 g (71 % der Theorie) 3-(4-Chlor-2-fluor-5-methoxycarbonyl-phenyl)-6-ethoxy-(1H,3H)-1,3,5-triazin-2,4-dion mit Schmelzpunkt 101-103 ° C.

Beispiel 4:

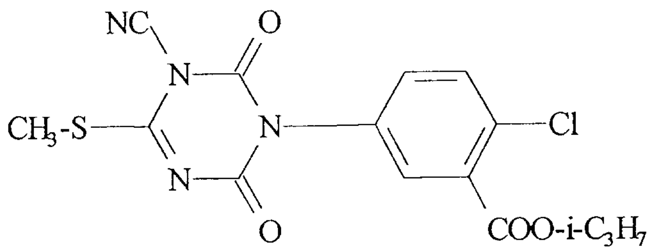

Zu einer Lösung von 2,5 g (0,007 Mol) 3-(4-Chlor-5-isopropoxycarbonyl-phenyl)-6-methylthio-(1H,3H)-1,3,5-triazin-2,4-dion und 0,9 g (0,008 Mol) Bromcyan in 60 ml Aceton gibt man bei 0°C bis 5°C unter Rühren 0,8 g (0,008 Mol) Triethylamin und rührt anschließend für 8 bis 16 Stunden bei Raumtemperatur. Zur Aufarbeitung wird die Reaktionsmischung in Wasser gegeben und mit Essigester extrahiert. Die organische Phase wird abgetrennt getrocknet und im Vakuum vom Lösungsmittel befreit.

Man erhält 0,9 g (34 % der Theorie) 3-(4-Chlor-5-isopropoxycarbonyl-phenyl)-1-cyano-6-methylthio-(1H,3H)-1,3,5-triazin-2,4-dion mit Schmelzpunkt 55-56°C.

Beispiel 5:

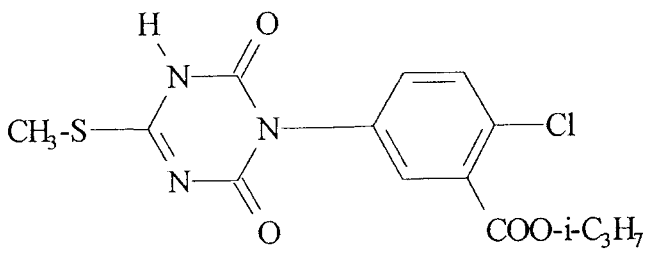

Zu einer Lösung von 18,2 g (0,047 Mol) N-(4-Chlor-3-isopropoxycarbonyl-phenyl)-N'-(1-methylthio-1-methoxycarbonylimino-methyl)-harnstoff in 250 ml Isopropanol gibt man bei Raumtemperatur 2,7 g (0,05 Mol) Natriummethylat und rührt anschliessend 16 Stunden bei Raumtemperatur. Zur Aufarbeitung wird das Lösungsmittel im Vakuum abdestilliert, der Rückstand in Eiswasser gegeben, mit Salzsäure angesäuert und mit Dichlormethan extrahiert. Die organische Phase wird mit verdünnter Salzsäure gewaschen, über Magnesiumsulfat getrocknet und im Vakuum eingeengt. Der Rückstand wird mit Methylcyclohexan verrieben, abgesaugt und getrocknet.

Man erhält 9,5 g (57 % der Theorie) 3-(4-Chlor-5-isopropoxycarbonyl-phenyl)-6-methylthio-(1H,3H)-1,3,5-triazin-2,4-dion mit Schmelzpunkt 114°C.

In entsprechender Weise und gemäß den allgemeinen Angaben zur Herstellung erhält man die folgenden 3-Aryl-triazin-2,4-dione der allgemeinen Formel (I):

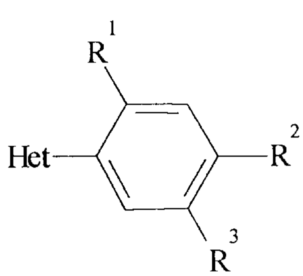

(I)

| Bsp. Nr. | Het | $R^1$ | $R^2$ | $R^3$ | physikalische Eigenschaften |
|---|---|---|---|---|---|
| 6 | 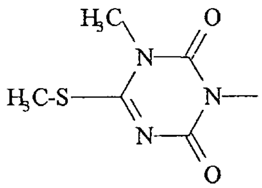 | H | Cl | $-COO-i-C_3H_7$ | Fp. 146°C |
| 7 | 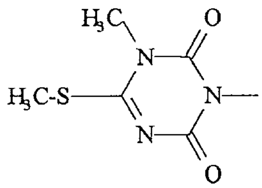 | H | Cl | $-COO-CH_3$ | Fp. 183°C |
| 8 | 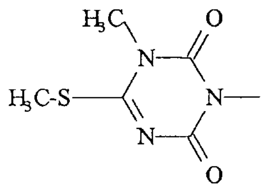 | F | Cl | $-O-CH(CH_3)-C\equiv CH$ | Fp. 121°C |
| 9 | 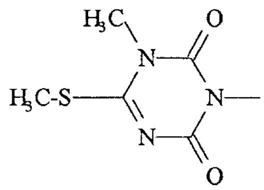 | F | Cl | $-COO-i-C_3H_7$ | Fp. 121°C |
| 10 | 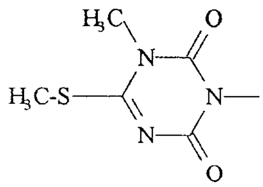 | F | Cl | $-COO-CH_3$ | Fp. 91-92°C |

| Bsp. Nr. | Het | $R^1$ | $R^2$ | $R^3$ | physikalische Eigenschaften |
|---|---|---|---|---|---|
| 11 | 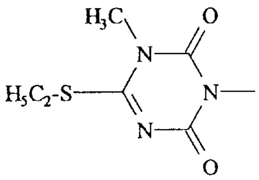 | F | Cl | $-COO-CH_3$ | Fp. 70-72°C |
| 12 | 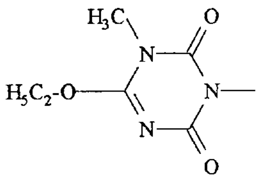 | H | Cl | $-COO-CH_3$ | Fp. 155°C |
| 13 | 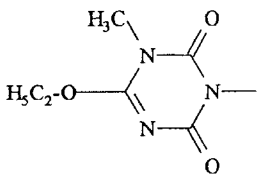 | F | Cl | $-COO-i-C_3H_7$ | Fp. 84°C |
| 14 | 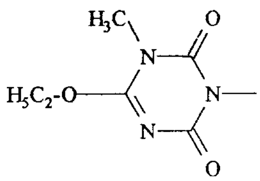 | H | Cl | $-COO-i-C_3H_7$ | Fp. 46-47°C |
| 15 | 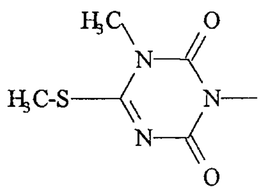 | F | CN | $-O-i-C_3H_7$ | Fp. 134°C |
| 16 | 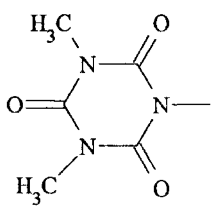 | H | Cl | $-COO-i-C_3H_7$ | Fp. 133°C |
| 17 | 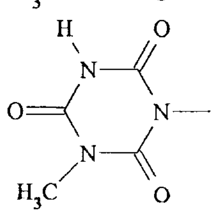 | H | Cl | $-COO-i-C_3H_7$ | Fp. 130°C |

| Bsp. Nr. | Het | $R^1$ | $R^2$ | $R^3$ | physikalische Eigenschaften |
|---|---|---|---|---|---|
| 18 | 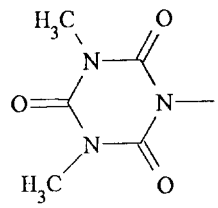 | F | Cl | $-COO-i-C_3H_7$ | Fp. 107-109°C |
| 19 | 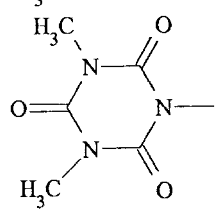 | F | Cl | $-COO-CH_3$ | Fp. 46-47°C |
| 20 | 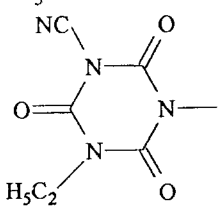 | F | Cl | $-COO-CH_3$ | Fp. 185°C |
| 21 | 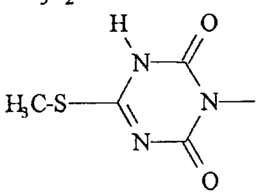 | H | Cl | $-COO-CH_3$ | Fp. 197°C |
| 22 | 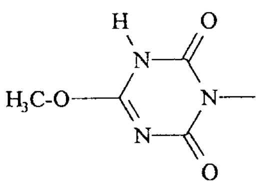 | H | Cl | $-COO-i-C_3H_7$ | Fp. 175°C |
| 23 | 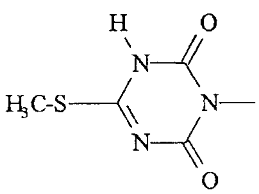 | F | Cl | $-O-CH(CH_3)-C{\equiv}CH$ | $^1$H-NMR*): 2,62 (s, 3H) |
| 24 | 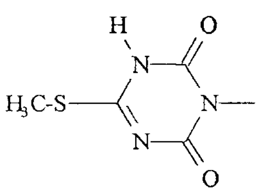 | F | Cl | $-COO-i-C_3H_7$ | Fp. 185-186°C |

| Bsp. Nr. | Het | $R^1$ | $R^2$ | $R^3$ | physikalische Eigenschaften |
|---|---|---|---|---|---|
| 25 | 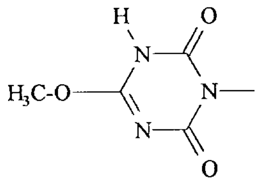 | F | Cl | $-COO-i-C_3H_7$ | Fp. 201-202°C |
| 26 | 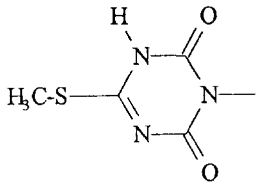 | F | Cl | $-COO-CH_3$ | Fp. 208-210°C |
| 27 | 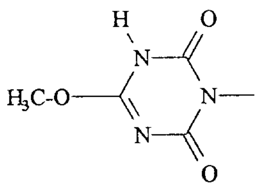 | F | Cl | $-COO-CH_3$ | Fp. 133-135°C |
| 28 | 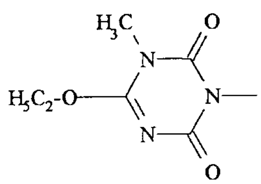 | F | Cl | $-O-CH(CH_3)-C{\equiv}CH$ | Fp. 53-55°C |
| 29 | 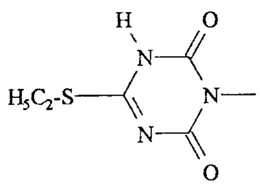 | F | Cl | $-COO-CH_3$ | Fp. 152°C |
| 30 | 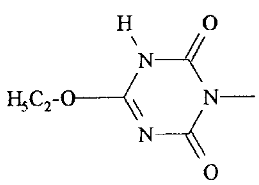 | F | Cl | $-COO-i-C_3H_7$ | Fp. 60-61°C |
| 31 | 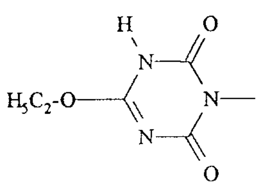 | H | Cl | $-COO-i-C_3H_7$ | Fp. 194-195°C |

| Bsp. Nr. | Het | $R^1$ | $R^2$ | $R^3$ | physikalische Eigenschaften |
|---|---|---|---|---|---|
| 32 | 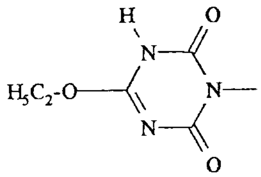 | H | Cl | $-COO-CH_3$ | Fp. 196-197°C |
| 33 | 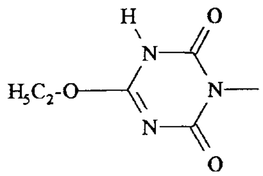 | F | Cl | $-O-C_2H_5$ | Fp. 104-105°C |
| 34 | 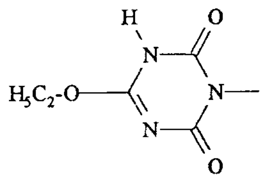 | F | Cl | $-O-CH(CH_3)-C{\equiv}CH$ | Fp. 169-170°C |
| 35 | 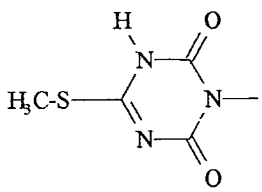 | F | CN | $-O-i-C_3H_7$ | Fp. 127°C |
| 36 | 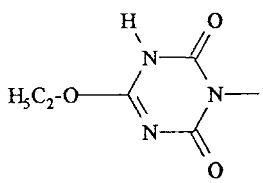 | F | CN | $-O-i-C_3H_7$ | Fp. 85°C |
| 37 | 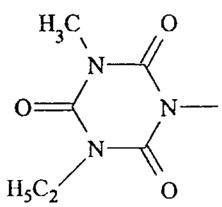 | H | Cl | $-COO-CH_3$ | Fp. 65-67°C |
| 38 | 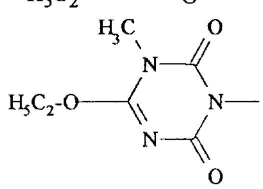 | F | Cl | $-O-C_2H_5$ | Fp. 114-115°C |

| Bsp. Nr. | Het | $R^1$ | $R^2$ | $R^3$ | physikalische Eigenschaften |
|---|---|---|---|---|---|
| 39 | 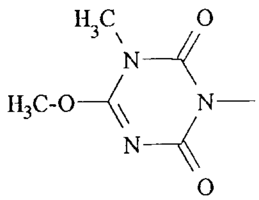 | H | Cl | $-COOCH_3$ | Fp. 142°C |
| 40 | 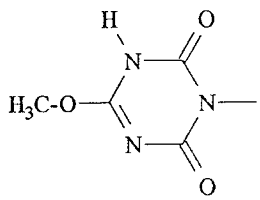 | H | Cl | $-COOCH_3$ | Fp. 169°C |
| 41 | 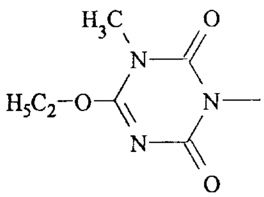 | F | CN | $-O-i-C_3H_7$ | Fp. 87°C |
| 42 | 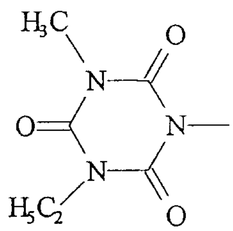 | H | Cl | $-COO-i-C_3H_7$ | Fp. 45°C |
| 43 | 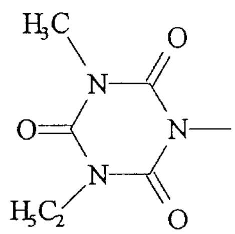 | H | Cl | $-COO-i-C_3H_7$ | Fp. 55-56°C |
| 44 | 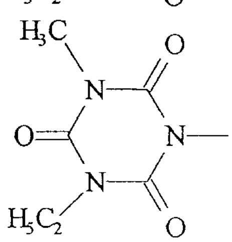 | F | Cl | $-O-C_2H_5$ | Fp. 96-97°C |

| Bsp. Nr. | Het | $R^1$ | $R^2$ | $R^3$ | physikalische Eigenschaften |
|---|---|---|---|---|---|
| 45 | 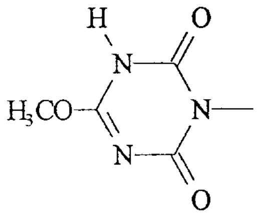 | F | Cl | $-O-C_2H_5$ | Fp. 133°C |
| 46 | 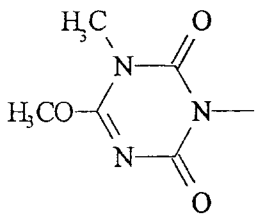 | F | Cl | $-O-C_2H_5$ | Fp. 134°C |
| 47 | 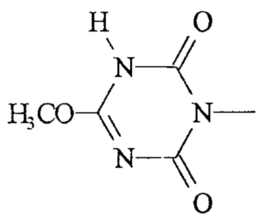 | H | Cl | $-O-CH(CH_3)-C{\equiv}CH$ | Fp. 147-148°C |
| 48 | 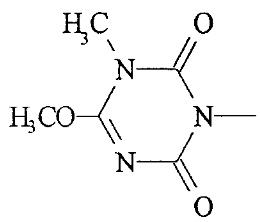 | F | Cl | $-O-CH(CH_3)-C{\equiv}CH$ | Fp. 45-46°C |
| 49 | 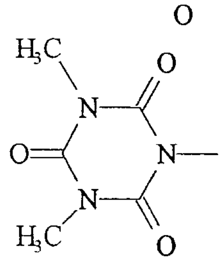 | F | Cl | $-O-C_2H_5$ | Fp. 139-140°C |
| 50 | 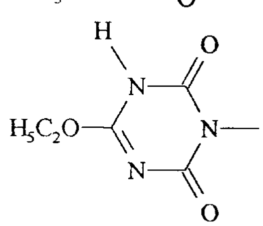 | F | Cl | $-S-CH(CH_3)-COOC_2H_5$ | Fp. 60-61°C |

| Bsp. Nr. | Het | $R^1$ | $R^2$ | $R^3$ | physikalische Eigenschaften |
|---|---|---|---|---|---|
| 51 | 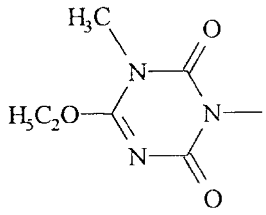 | F | Cl | -S-CH($CH_3$)-$COOC_2H_5$ | $^1$H-NMR*): 4,62 (q, 2H) |

*) Die $^1$H-NMR-Spektren wurden in Deuterochloroform ($CDCl_3$) oder Hexadeuterodimethylsulfoxid (DMSO-$d_6$) mit Tetramethylsilan (TMS) als innerem Standard aufgenommen. Angegeben ist die chemische Verschiebung als δ-Wert in ppm.

**Herstellung derAusgangsverbindungen:**

Beispiel II-1:

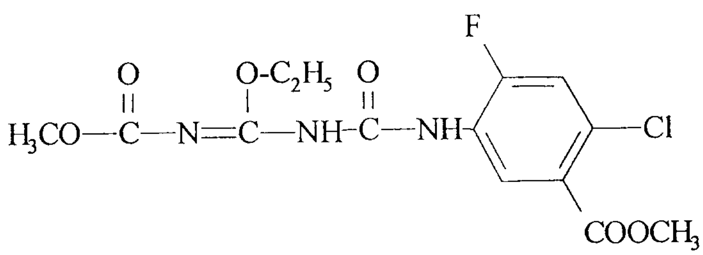

Zu einer Lösung von 10,1 g (0,05 Mol) 5-Amino-2-chlor-4-fluor-benzoesäure-methylester in 150 ml Essigester gibt man bei 70 ° C unter Rühren 10 g (0,05 Mol) Chlorameisensäure-trichlormethylester (Diphosgen) und erhitzt anschließend für 2 Stunden auf Rückflußtemperatur. Anschließend wird das Lösungsmittel abdestilliert, der Rückstand in 100 ml Dichlormethan aufgenommen, mit 7,3 g (0,05 Mol) N-(1-Ethoxy-1-imino-methyl)-carbaminsäuremethylester versetzt und 12 bis 16 Stunden bei Raumtemperatur gerührt. Zur Aufarbeitung wird ausgefallener Niederschlag abgesaugt, das Filtrat im Vakuum eingeengt und der Rückstand aus Isopropanol umkristallisiert.

Man erhält 5,3 g (28 % der Theorie) N-(4-Chlor-2-fluor-5-methoxycarbonylphenyl)-N'-(1-ethoxy-1-methoxycarbonylimino-methyl)-harnstoff mit Schmelzpunkt 105-107 ° C.

Beispiel II-2:

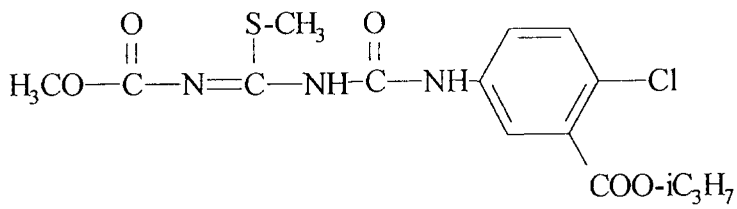

Zu einer Lösung von 12,9 g (0,06 Mol) 5-Amino-2-chlor-benzoesäure-isopropylester in 200 ml Essigester gibt man bei 70 °C unter Rühren 12 g (0,06 Mol) Chlorameisensäure-trichlormethylester (Diphosgen) und erhitzt anschließend für 2 Stunden auf Rückflußtemperatur. Anschließend wird das Lösungsmittel abdestilliert, der Rückstand in 100 ml Dichlormethan aufgenommen, mit 8,9 g (0,06 Mol) N-(1-Methylthio-1-imino-methyl)-carbaminsäuremethylester versetzt und 16 Stunden bei Raumtemperatur gerührt. Zur Aufarbeitung wird das Lösungsmittel im Vakuum abdestilliert, der verbleibende Rückstand mit Methylcyclohexan verrieben, abgesaugt und getrocknet.

Man erhält 22,6 g (97 % der Theorie) N-(4-Chlor-5-isopropoxycarbonyl-phenyl)-N'-(1-methylthio-1-methoxycarbonylimino-methyl)-harnstoff mit Schmelzpunkt 93-94 °C.

In entsprechender Weise und gemäß den allgemeinen Angaben zur Herstellung erhält man die folgenden substituierten Phenylharnstoff-Derivate der allgemeinen Formel (II):

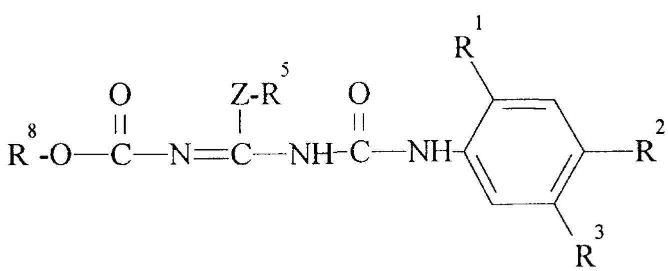

(II)

| Bsp. Nr. | $R^1$ | $R^2$ | $R^3$ | $R^5$ | $R^8$ | Z | Schmelzpunkt / °C |
|---|---|---|---|---|---|---|---|
| II-3 | H | Cl | $-COO-CH_3$ | $CH_3$ | $CH_3$ | S | 158 |
| II-4 | H | Cl | $-COO-i-C_3H_7$ | $CH_3$ | $CH_3$ | O | 108 |
| II-5 | F | Cl | $-COO-i-C_3H_7$ | $CH_3$ | $CH_3$ | S | 129-131 |
| II-6 | F | Cl | $-COO-i-C_3H_7$ | $CH_3$ | $CH_3$ | O | 100-101 |
| II-7 | F | Cl | $-COO-CH_3$ | $CH_3$ | $CH_3$ | S | 167-169 |
| II-8 | F | Cl | $-COO-CH_3$ | $CH_3$ | $CH_3$ | O | 159-160 |
| II-9 | F | Cl | $-COO-CH_3$ | $C_2H_5$ | $CH_3$ | S | 115-116 |
| II-10 | H | Cl | $-COO-i-C_3H_7$ | $C_2H_5$ | $CH_3$ | O | 110-111 |
| II-11 | H | Cl | $-COO-CH_3$ | $C_2H_5$ | $CH_3$ | O | 106-107 |
| II-12 | F | Cl | $-COO-i-C_3H_7$ | $C_2H_5$ | $CH_3$ | O | 75-76 |
| II-13 | F | Cl | $-O-CH(CH_3)-C\equiv CH$ | $C_2H_5$ | $CH_3$ | O | 81-82 |
| II-14 | F | Cl | $-O-C_2H_5$ | $C_2H_5$ | $CH_3$ | O | 75-77 |
| II-15 | F | CN | $-O-i-C_3H_7$ | $CH_3$ | $CH_3$ | S | 129 |
| II-16 | F | CN | $-O-i-C_3H_7$ | $C_2H_5$ | $CH_3$ | O | 122-123 |
| II-17 | F | Cl | $-O-CH(CH_3)-C\equiv CH$ | $CH_3$ | $CH_3$ | S | |
| II-18 | F | Cl | $-O-C_2H_5$ | $CH_3$ | $CH_3$ | O | 92-94 |
| II-19 | F | Cl | $O-CH(CH_3)C\equiv CH$ | $CH_3$ | $CH_3$ | O | 74-75 |
| II-20 | F | Cl | $-S-CH(CH_3)-COOC_2H_5$ | $C_2H_5$ | $CH_3$ | O | Öl |

Anwendungsbeispiele:

In den folgenden Anwendungsbeispielen wurde die nachstehend aufgeführte Verbindung als Vergleichssubstanz eingesetzt:

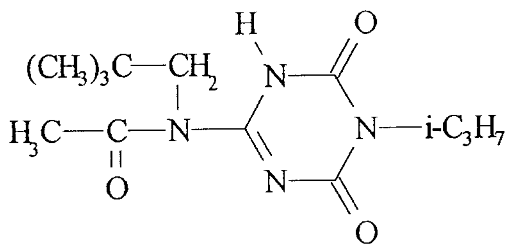

(A)

3-Isopropyl-6-[N-acetyl-N-(2,2-dimethyl-1-propyl)-amino]-(3H,5H)-1,3,5-triazin-2,4-dion
(vergl. z.B. DE 26 03 180)

Beispiel A:

**Pre-emergence-Test**

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoftzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigt in diesem Test z.B. die Verbindung gemäß Herstellungsbeispielen 7.

Beipiel B:

**Post-emergence-Test**

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffbereitung spritzt man Testpflanzen, welche eine Höhe von 5 bis 15 cm haben so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100% = totale Vernichtung

Eine deutliche Überlegenheit in der Wirksamkeit ebenso wie in der Nutzpflanzenselektivität gegenüber dem Stand der Technik zeigt in diesem Test z.B. die Verbindung gemäß Herstellungsbeispielen 6.

Beipiel C:

**Entlaubung und Austrocknung der Blätter bei Baumwolle**

Lösungsmittel: 30 Gewichtsteile Dimethyformamid
Emulgator: 1 Gewichtsteil Polyoxyethylen-Sorbitan-Monolaureat

Zur Herstellung einer zweckmäßigen Wirkstoftzubereitung vermischt man ein Gewichtsteil Wirkstoff mit den angegebenen Mengen Lösungsmittel und Emulgator und füllt das Konzentrat mit Wasser auf die gewünschte Konzentration auf.

Baumwollpflanzen werden im Gewächshaus bis zur vollen Entfaltung des fünften Folgeblattes angezogen. In diesem Stadium werden die Pflanzen tropfnass mit den Wirkstoffzubereitungen besprüht. Nach einer Woche werden Blattfall und Austrocknung der Blätter im Vergleich zur Entwicklung der unbehandelten Kontrolle bonitiert.

Eine deutliche Überlegenheit in der Wirksamkeit gegenüber der unbehandelten Kontrolle zeigt in diesem Test z.B. die Verbindung gemäß Herstellungsbeispiel 6.

**Patentansprüche**

**1.** Neue 3-Aryl-triazin-2,4-dione der allgemeinen Formel (I),

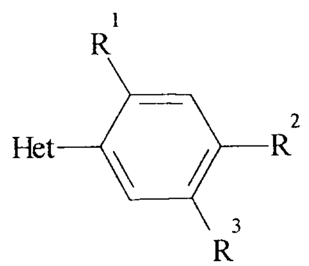

(I)

in welcher

Het für einen Rest der Formel

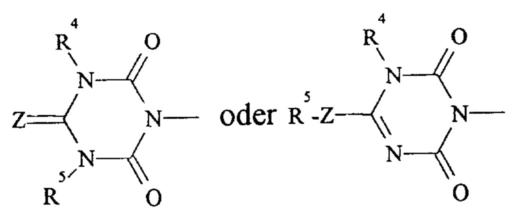

steht,

- $R^1$ für Wasserstoff oder Halogen steht,
- $R^2$ für Halogen, Cyano oder Nitro steht und
- $R^3$ für einen Rest der Formel $-X-R^6$ oder $-CO-X-R^7$ wobei
- $R^4$ für Wasserstoff, Cyano, Alkyl, Alkenyl, Alkinyl oder Halogenalkyl steht,
- $R^5$ für Alkyl, Alkenyl oder Alkinyl steht,
- $R^6$ für Wasserstoff oder für gegebenenfalls substituiertes Alkyl steht,
- $R^7$ für Wasserstoff oder für gegebenenfalls substituiertes Alkyl steht,
- X für Sauerstoff oder Schwefel steht und
- Z für Sauerstoff oder Schwefel steht.

**2.** 3-Aryl-triazin-2,4-dione der allgemeinen Formel (I) gemäß Anspruch 1, dadurch gekennzeichnet, daß Het für einen Rest der Formel

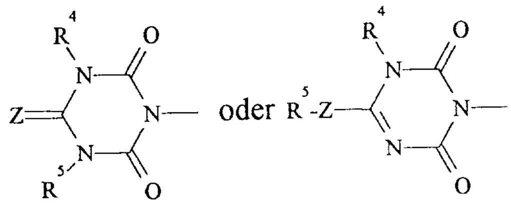

steht,

$R^1$ für Wasserstoff, Fluor, Chlor Brom oder Iod steht,

$R^2$ für Fluor, Chlor Brom, Iod, Cyano oder Nitro steht,

$R^3$ für einen Rest der Formel $-X-R^6$ oder $-CO-X-R^7$ steht, wobei

$R^4$ für Wasserstoff, Cyano, für geradkettiges oder verzweigtes Alkyl mit 1 bis 8 Kohlenstoffatomen, für geradkettiges oder verzweigtes Alkenyl mit 2 bis 8 Kohlenstoffatomen, für geradkettiges oder verzweigtes Alkinyl mit 2 bis 8 Kohlenstoffatomen oder für geradkettiges oder verzweigtes Halogenalkyl mit 1 bis 6 Kohlenstoffatomen und 1 bis 13 gleichen oder verschiedenen Halogenatomen - insbesondere Fluor, Chlor, Brom und/oder Iod - steht,

$R^5$ für geradkettiges oder verzweigtes Alkyl mit 1 bis 8 Kohlenstoffatomen, für geradkettiges oder verzweigtes Alkenyl mit 2 bis 8 Kohlenstoffatomen oder für geradkettiges oder verzweigtes Alkinyl mit 2 bis 8 Kohlenstoffatomen steht,

$R^6$ jeweils für Wasserstoff, für geradkettiges oder verzweigtes Alkyl mit 1 bis 8 Kohlenstoffatomen, für geradkettiges oder verzweigtes Halogenalkyl mit 1 bis 6 Kohlenstoffatomen und 1 bis 13 gleichen oder verschiedenen Halogenatomen - insbesondere Fluor, Chlor, Brom und/oder Iod-, für jeweils geradkettiges oder verzweigtes Alkoxyalkyl, Alkoxycarbonylalkyl, Alkylcarbonyloxyalkyl, Alkylthiocarbonylalkyl oder Alkylcarbonylthioalkyl mit jeweils 1 bis 6 Kohlenstoffatomen in den einzelnen Alkylteilen oder für gegebenenfalls einfach oder mehrfach, gleich oder verschieden substituiertes Arylalkyl mit 6 oder 10 Kohlenstoffatomen im Arylteil und 1 bis 6 Kohlenstoffatomen im geradkettigen oder verzweigten Alkylteil stehen, wobei als Arylsubstituenten infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl und/oder geradkettiges oder verzweigtes Alkoxy und/oder geradkettiges oder verzweigtes Halogenalkyl und/oder geradkettiges oder verzweigtes Halogenalkoxy mit jeweils 1 bis 4 Kohlenstoffatomen und gegebenenfalls 1 bis 9 gleichen oder verschiedenen Halogenatomen substituiertes Phenyl,

$R^7$ jeweils für Wasserstoff, für geradkettiges oder verzweigtes Alkyl mit 1 bis 8 Kohlenstoffatomen, für geradkettiges oder verzweigtes Halogenalkyl mit 1 bis 6 Kohlenstoffatomen und 1 bis 13 gleichen oder verschiedenen Halogenatomen - insbesondere Fluor, Chlor, Brom und/oder Iod -, für jeweils geradkettiges oder verzweigtes Alkoxyalkyl, Alkoxycarbonylalkyl, Alkylcarbonyloxyalkyl, Alkylthiocarbonylalkyl oder Alkylcarbonylthioalkyl mit jeweils 1 bis 6 Kohlenstoffatomen in den einzelnen Alkylteilen oder für gegebenenfalls einfach oder mehrfach, gleich oder verschieden substituiertes Arylalkyl mit 6 oder 10 Kohlenstoffatomen im Arylteil und 1 bis 6 Kohlenstoffatomen im geradkettigen oder verzweigten Alkylteil stehen, wobei als Arylsubstituenten infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl und/oder geradkettiges oder verzweigtes Alkoxy

und/oder geradkettiges oder verzweigtes Halogenalkyl und/oder geradkettiges oder verzweigtes Halogenalkoxy mit jeweils 1 bis 4 Kohlenstoffatomen und gegebenenfalls 1 bis 9 gleichen oder verschiedenen Halogenatomen substituiertes Phenyl,

X für Sauerstoff oder Schwefel steht und

Z für Sauerstoff oder Schwefel steht.

**3.** 3-Aryl-triazin-2,4-dione der allgemeinen Formel (I), gemäß Anspruch 1, dadurch gekennzeichnet, daß Het für einen Rest der Formel

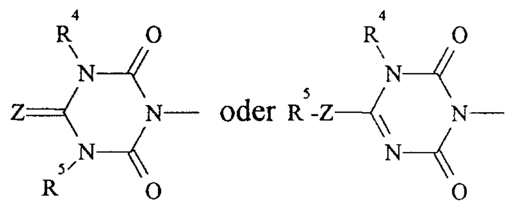

steht,

$R^1$ für Wasserstoff, Fluor, Chlor oder Brom steht,

$R^2$ für Fluor, Chlor, Brom, Cyano oder Nitro steht,

$R^3$ für einen Rest der Formel $-X-R^6$ oder $-CO-X-R^7$ steht, wobei

$R^4$ für Wasserstoff, Cyano, für geradkettiges oder verzweigtes Alkyl mit 1 bis 6 Kohlenstoffatomen, für geradkettiges oder verzweigtes Alkenyl mit 2 bis 6 Kohlenstoffatomen, für geradkettiges oder verzweigtes Alkinyl mit 2 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom - steht,

$R^5$ für geradkettiges oder verzweigtes Alkyl mit 1 bis 6 Kohlenstoffatomen, für geradkettiges oder verzweigtes Alkenyl mit 2 bis 6 Kohlenstoffatomen oder für geradkettiges oder verzweigtes Alkinyl mit 2 bis 6 Kohlenstoffatomen steht,

$R^6$ für Wasserstoff, für geradkettiges oder verzweigtes Alkyl mit 1 bis 6 Kohlenstoffatomen, für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom für jeweils geradkettiges oder verzweigtes Alkoxyalkyl, Alkoxycarbonylalkyl, Alkylcarbonyloxyalkyl, Alkylthiocarbonylalkyl oder Alkylcarbonylthioalkyl mit jeweils 1 bis 4 Kohlenstoffatomen in den einzelnen Alkylteilen oder für gegebenenfalls einfach bis fünffach, gleich oder verschieden substituiertes Phenylalkyl mit 1 bis 4 Kohlenstoffatomen im geradkettigen oder verzweigten Alkylteil stehen, wobei als Phenylsubstituenten infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach oder mehrfach, gleich oder verschieden durch Halogen und/oder geradkettiges oder verzweigtes Alkyl und/oder geradkettiges oder verzweigtes Alkoxy und/oder geradkettiges oder verzweigtes Halogenalkyl und/oder geradkettiges oder verzweigtes Halogenalkoxy mit jeweils 1 bis 3 Kohlenstoffatomen und gegebenenfalls 1 bis 7 gleichen oder verschiedenen Halogenatomen substituiertes Phenyl,

$R^7$ für Wasserstoff, für geradkettiges oder verzweigtes Alkyl mit 1 bis 6 Kohlenstoffatomen, für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom -, für jeweils geradkettiges oder verzweigtes Alkoxyalkyl, Alkoxycarbonylalkyl, Alkylcarbonyloxyalkyl, Alkylthiocarbonylalkyl oder Alkylcarbonylthioalkyl mit jeweils 1 bis 4 Kohlenstoffatomen in den einzelnen Alkylteilen oder für gegebenenfalls einfach bis fünffach, gleich oder verschieden substituiertes Phenylalkyl mit 1 bis 4 Kohlenstoffatomen im geradkettigen oder verzweigten Alkylteil stehen, wobei als Phenylsubstituenten infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 3 Kohlenstoffatomen, jeweils geradkettiges oder

verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach oder mehrfach, gleich oder verschieden durch Halogen und/oder geradkettiges oder verzweigtes Alkyl und/oder geradkettiges oder verzweigtes Alkoxy und/oder geradkettiges oder verzweigtes Halogenalkyl und/oder geradkettiges oder verzweigtes Halogenalkoxy mit jeweils 1 bis 3 Kohlenstoffatomen und gegebenenfalls 1 bis 7 gleichen oder verschiedenen Halogenatomen substituiertes Phenyl,

X für Sauerstoff oder Schwefel steht und

Z für Sauerstoff oder Schwefel steht.

**4.** 3-Aryl-triazin-2,4-dione der allgemeinen Formel (I) gemäß Anspruch 1, dadurch gekennzeichnet, daß Het für einen Rest der Formel

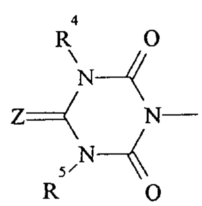

oder

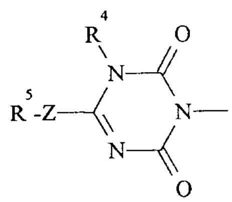

steht,

$R^1$ für Wasserstoff, Fluor oder Chlor steht,

$R^2$ für Fluor, Chlor, Cyano oder Nitro steht,

$R^3$ für einen Rest der Formel -X-$R^6$ oder -CO-X-$R^7$ steht, wobei

$R^4$ für Wasserstoff, Cyano, für geradkettiges oder verzweigtes Alkyl mit 1 bis 4 Kohlenstoffatomen, für geradkettiges oder verzweigtes Alkenyl mit 2 bis 4 Kohlenstoffatomen, für geradkettiges oder verzweigtes Alkinyl mit 2 bis 4 Kohlenstoffatomen oder für geradkettiges oder verzweigtes Halogenalkyl mit 1 bis 3 Kohlenstoffatomen und 1 bis 7 gleichen oder verschiedenen Halogenatomen - insbesondere Fluor, Chlor und/oder Brom - steht,

$R^5$ für geradkettiges oder verzweigtes Alkyl mit 1 bis 4 Kohlenstoffatomen, für geradkettiges oder verzweigtes Alkenyl mit 2 bis 4 Kohlenstoffatomen oder für geradkettiges oder verzweigtes Alkinyl mit 2 bis 4 Kohlenstoffatomen steht,

$R^6$ für Wasserstoff, für geradkettiges oder verzweigtes Alkyl mit 1 bis 4 Kohlenstoffatomen, für geradkettiges oder verzweigtes Halogenalkyl mit 1 bis 3 Kohlenstoffatomen und 1 bis 7 gleichen oder verschiedenen Halogenatomen - insbesondere Fluor, Chlor oder Brom -, für jeweils geradkettiges oder verzweigtes Alkoxyalkyl, Alkoxycarbonylalkyl, Alkylcarbonyloxyalkyl, Alkylthiocarbonylalkyl oder Alkylcarbonylthioalkyl mit jeweils 1 bis 3 Kohlenstoffatomen in den einzelnen Alkylteilen oder für gegebenenfalls einfach bis dreifach, gleich oder verschieden substituiertes Phenylalkyl mit 1 bis 2 Kohlenstoffatomen im Alkylteil stehen, wobei als Phenylsubstituenten infrage kommen:
Fluor, Chlor, Brom, Cyano, Nitro, Methyl, Ethyl n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Ethylsulfinyl, Methylsulfonyl, Ethylsulfonyl, Trifluormethyl, Difluormethyl, Trifluormethoxy, Difluormethoxy, Trifluormethylthio, Trifluormethylsulfinyl, Trifluormethylsulfonyl, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl, Ethyl, n- oder i-Propyl, Methoxy, Ethoxy, Trifluormethyl und/oder Trifluormethoxy substituiertes Phenyl,

$R^7$ für Wasserstoff, für geradkettiges oder verzweigtes Alkyl mit 1 bis 4 Kohlenstoffatomen, für geradkettiges oder verzweigtes Halogenalkyl mit 1 bis 3 Kohlenstoffatomen und 1 bis 7 gleichen oder verschiedenen Halogenatomen - insbesondere Fluor, Chlor oder Brom -, für jeweils geradkettiges oder verzweigtes Alkoxyalkyl, Alkoxycarbonylalkyl, Alkylcarbonyloxyalkyl, Alkylthiocarbonylalkyl oder Alkylcarbonylthioalkyl mit jeweils 1 bis 3 Kohlenstoffatomen in den einzelnen Alkylteilen oder für gegebenenfalls einfach bis dreifach, gleich oder verschieden substituiertes Phenylalkyl mit 1 bis 2 Kohlenstoffatomen im Alkylteil stehen, wobei als

Phenylsubstituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Ethylsulfinyl, Methylsulfonyl, Ethylsulfonyl, Trifluormethyl, Difluormethyl, Trifluormethoxy, Difluormethoxy, Trifluormethylthio, Trifluormethylsulfinyl, Trifluormethylsulfonyl, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl, Ethyl, n- oder i-Propyl, Methoxy, Ethoxy, Trifluormethyl und/oder Trifluormethoxy substituiertes Phenyl,

X für Sauerstoff oder Schwefel steht und

Z für Sauerstoff oder Schwefel steht.

**5.** Verfahren zur Herstellung von 3-Aryl-triazin-2,4-dionen der allgemeinen Formel (I),

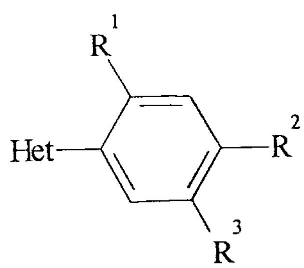

(I)

in welcher

Het für einen Rest der Formel

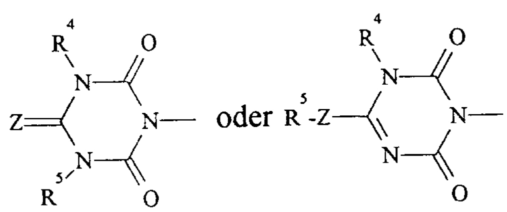

steht,

$R^1$ für Wasserstoff oder Halogen steht,

$R^2$ für Halogen, Cyano oder Nitro steht und

$R^3$ für einen Rest der Formel $-X-R^6$ oder $-CO-X-R^7$ steht, wobei

$R^4$ für Wasserstoff, Cyano, Alkyl, Alkenyl, Alkinyl oder Halogenalkyl steht,

$R^5$ für Alkyl, Alkenyl oder Alkinyl steht,

$R^6$ für Wasserstoff oder für gegebenenfalls substituiertes Alkyl steht,

$R^7$ für Wasserstoff oder für gegebenenfalls substituiertes Alkyl steht,

X für Sauerstoff oder Schwefel steht und

Z für Sauerstoff oder Schwefel steht,

dadurch gekennzeichnet, daß man

substituierte Phenylharnstoff-Derivate der allgemeinen Formel (II),

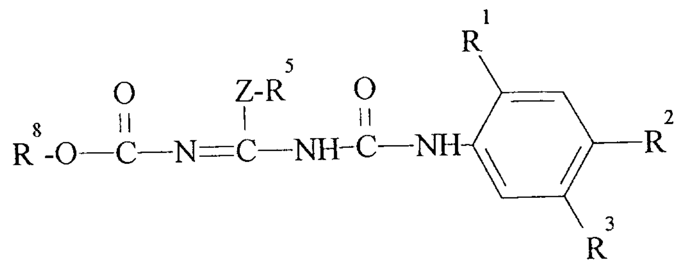

(II)

in welcher

$R^1$, $R^2$, $R^3$, $R^5$ und Z die oben angegebenen Bedeutungen haben und

$R^8$ für Alkyl oder Aryl steht,

gegebenenfalls in Gegenwart eines Verdünnungsmittels und gegebenenfalls in Gegenwart eines Reaktionshilfsmittels cyclisiert und gegebenenfalls in einer anschließendenden 2.Stufe die so erhältlichen 3-Aryl-(1H)-triazin-2,4-dione der Formel (Ia),

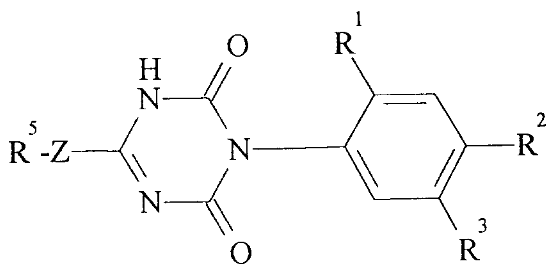

(Ia)

in welcher

$R^1$, $R^2$, $R^3$, $R^5$ und Z die die oben angegebenen Bedeutungen haben mit Alkylierungsmitteln der Formel (III),

$R^{4-1}$-E (III)

in welcher

$R^{4-1}$ für Alkyl, Alkenyl, Alkinyl oder Halogenalkyl steht und

E für eine elektronenanziehende Abgangsgruppe steht,

oder mit Bromcyan gegebenenfalls in Gegenwart eines Verdünnungsmittels und gegebenenfalls in Gegenwart eines Reaktionshilfsmittels umsetzt und gegebenenfalls in einer anschließendenden 3.Stufe die so erhältlichen 3-Aryl-triazin-2,4-dione der Formel (Ib),

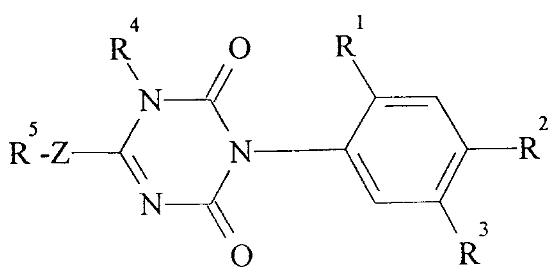

(Ib)

in welcher

$R^1$, $R^2$, $R^3$, $R^4$, $R^5$ und Z die die oben angegebenen Bedeutungen haben,

gegebenenfalls in Gegenwart eines Verdünnungsmittels und gegebenenfalls in Gegenwart eines Reaktionshilfsmittels thermisch isomerisiert.

6. Verfahren zur Bekämpfung von unerwünschten Pflanzen, dadurch gekennzeichnet, daß man 3-Aryl-triazin-2,4-dione der Formel (I) gemäß den Ansprüchen 1 bis 5 auf unerwünschte Pflanzen und/oder ihren Lebensraum einwirken läßt.

7. Verwendung von 3-Aryltriazin-2,4-dionender Formel (I) gemäß den Ansprüchen 1 bis 5 zur Bekämpfung von unerwünschten Pflanzen.

8. Verfahren zur Herstellung von herbiziden und fungiziden Mitteln, dadurch gekennzeichnet, daß man 3-Aryl-triazin-2,4-dione der Formel (I) gemäß den Ansprüchen 1 bis 5 mit Streckmitteln und/oder oberflächenaktiven Substanzen vermischt

9. Herbizide Mittel, gekennzeichnet durch einen Gehalt an 3-Aryl-triazin-2,4-dion der Formel (I) gemäß den Ansprüchen 1 bis 5.

10. Phenylharnstoff-Derivate der allgemeinen Formel (II),

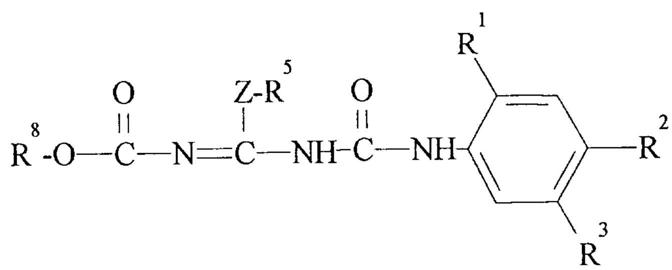

(II)

in welcher

- $R^1$ für Wasserstoff oder Halogen steht,
- $R^2$ für Halogen, Cyano oder Nitro steht und
- $R^3$ für einen Rest der Formel $-X-R^6$ oder $-CO-X-R^7$ wobei
- $R^5$ für Alkyl, Alkenyl oder Alkinyl steht,
- $R^6$ für Wasserstoff oder für gegebenenfalls substituiertes Alkyl steht,
- $R^7$ für Wasserstoff oder für gegebenenfalls substituiertes Alkyl steht,
- X für Sauerstoff oder Schwefel steht und
- Z für Sauerstoff oder Schwefel steht,

und

- $R^8$ für Alkyl oder Aryl steht.